# EUROPEAN PATENT APPLICATION

(11) **EP 4 137 200 A1**
(43) Date of publication of application: **22.02.2023**
(21) Application number: 21826635.1
(22) Date of filing: 19.04.2021
(51) Int. Cl.: A61N 1/39, A61B 5/1455, A61B 5/33

(54) **DEFIBRILLATION DEVICE AND METHOD**

(30) Priority: 19.06.2020 JP 2020106189
(71) Applicant: Hamamatsu Photonics K.K., Hamamatsu-shi, Shizuoka 435-8558 (JP)
(72) Inventor: KAMADA, Tsuyoshi, Hamamatsu-shi, Shizuoka 435-8558 (JP); KANO, Hitoshi, Hamamatsu-shi, Shizuoka 435-8558 (JP)
(74) Representative: Hoffmann Eitle
(86) International application number: PCT/JP2021/015880
(87) International publication number: WO 2021/256074

(57) **Abstract**

A defibrillator 1 is an apparatus for defibrillation, and includes an oxygen saturation (TOI) measurement unit 11 for acquiring a numerical value related to an oxygen saturation of a patient, an electrocardiogram (ECG) measurement unit 6 for measuring an electrocardiogram of the patient in order to determine whether an electrical shock is required for the patient, and a control unit 12 for starting measurement of the electrocardiogram of the patient in the electrocardiogram measurement unit 6 on the condition that the numerical value acquired in the oxygen saturation measurement unit 11 exceeds a threshold value. Thus, an apparatus and a method for defibrillation capable of increasing a success rate of defibrillation by an electrical shock and reducing the number of times of electrocardiogram analysis and the electrical shock can be realized.

## Description

### Technical Field

The present disclosure relates to an apparatus and a method for defibrillation.

### Background Art

Patent Document 1 discloses an apparatus for assisting a rescuer in providing cardio pulmonary resuscitation (CPR) to a patient. The apparatus includes at least one sensor of a pulse sensor for measuring a pulse rate of the patient and a SpO₂ sensor for measuring an amount of oxygen in blood, an electronic device for processing an output of the sensor and determining one or more actions to be performed by the rescuer in order to improve the current CPR, and an alerting device for notifying the rescuer of the one or more actions.

### Citation List

### Patent Literature

Patent Document 1: Japanese Patent Application Laid-Open Publication No. 2005-46606

### Summary of Invention

### Technical Problem

In the cardio pulmonary resuscitation, an electrical shock is applied for removing an arrhythmia such as a ventricular fibrillation (VF) of the patient. Before the electrical shock is applied, an electrocardiogram analysis is performed in order to confirm the arrhythmia state of the patient. For example, an automated external defibrillator (AED) automatically performs a series of operations from the electrocardiogram analysis to the electrical shock.

However, the electrocardiogram analysis and charging for the electrical shock require a certain amount of time, and further, chest compression cannot be performed when the electrocardiogram analysis and the charging are performed. Therefore, a blood oxygen concentration once increased by the chest compression gradually decreases during the electrocardiogram analysis and the charging. Thus, when the electrocardiogram analysis and the electrical shock are repeated, the blood oxygen concentration increased by the chest compression is decreased each time. As a result, the increase in blood oxygen concentration becomes slow, and it takes time for the cardio pulmonary resuscitation.

An object of an embodiment is to provide an apparatus and a method for defibrillation capable of increasing a success rate of defibrillation by an electrical shock and reducing the number of times of electrocardiogram analysis and the electrical shock.

### Solution to Problem

An embodiment is an apparatus for defibrillation. The apparatus for defibrillation includes an oxygen saturation measurement unit for acquiring a numerical value related to an oxygen saturation of a patient; an electrocardiogram measurement unit for measuring an electrocardiogram of the patient in order to determine whether an electrical shock is required for the patient; and a control unit for starting measurement of the electrocardiogram of the patient in the electrocardiogram measurement unit on the condition that the numerical value acquired in the oxygen saturation measurement unit exceeds a threshold value.

An embodiment is a method for defibrillation. The method for defibrillation includes a step of starting acquisition of a numerical value related to an oxygen saturation of a patient; a step of measuring an electrocardiogram of the patient on the condition that the numerical value related to the oxygen saturation exceeds a threshold value; and a step of determining whether an electrical shock is required for the patient based on a measurement result of the electrocardiogram.

In the above apparatus and the method, the electrocardiogram for determining whether the electrical shock is required is measured on the condition that the oxygen saturation of the patient exceeds the threshold value. As described later, the present inventors have found that the oxygen saturation of the patient affects success or failure of the defibrillation by the electrical shock.

When the oxygen saturation of the patient exceeds the certain threshold value, a success rate of the defibrillation by the electrical shock increases significantly. Therefore, when the oxygen saturation exceeds the threshold value, the electrocardiogram analysis and the electrical shock are performed, and when the oxygen saturation does not exceed the threshold value, the chest compression is preferentially performed without performing the electrocardiogram analysis and the electrical shock. As a result, it is possible to increase the success rate of the defibrillation by the electrical shock, and reduce the number of times of the electrocardiogram analysis and the electrical shock.

### Advantageous Effects of Invention

According to the embodiments, it is possible to provide an apparatus and a method for defibrillation capable of increasing a success rate of defibrillation by an electrical shock and reducing the number of times of electrocardiogram analysis and the electrical shock.

### Brief Description of Drawings

[FIG. 1] FIG. 1 is a functional block diagram illustrating a configuration of a defibrillator according to an embodiment.
[FIG. 2] FIG. 2 is a diagram illustrating a specific configuration example of a TOI measurement unit.
[FIG. 3] FIG. 3 is a flowchart illustrating an operation of the defibrillator and a method for defibrillation.
[FIG. 4] FIG. 4 is a graph showing a result of actual measurement of a TOI in cardio pulmonary resuscitation.
[FIG. 5] FIG. 5 is a graph showing a result of actual measurement of the TOI in the cardio pulmonary resuscitation.
[FIG. 6] FIG. 6 is a graph showing a result of actual measurement of the TOI in the cardio pulmonary resuscitation.
[FIG. 7] FIG. 7 is a graph showing a result of actual measurement of the TOI in the cardio pulmonary resuscitation.

### Description of Embodiments

Hereinafter, embodiments of an apparatus and a method for defibrillation will be described in detail with reference to the accompanying drawings. In the description of the drawings, the same elements will be denoted by the same reference signs, and redundant description will be omitted. The present invention is not limited to these examples.

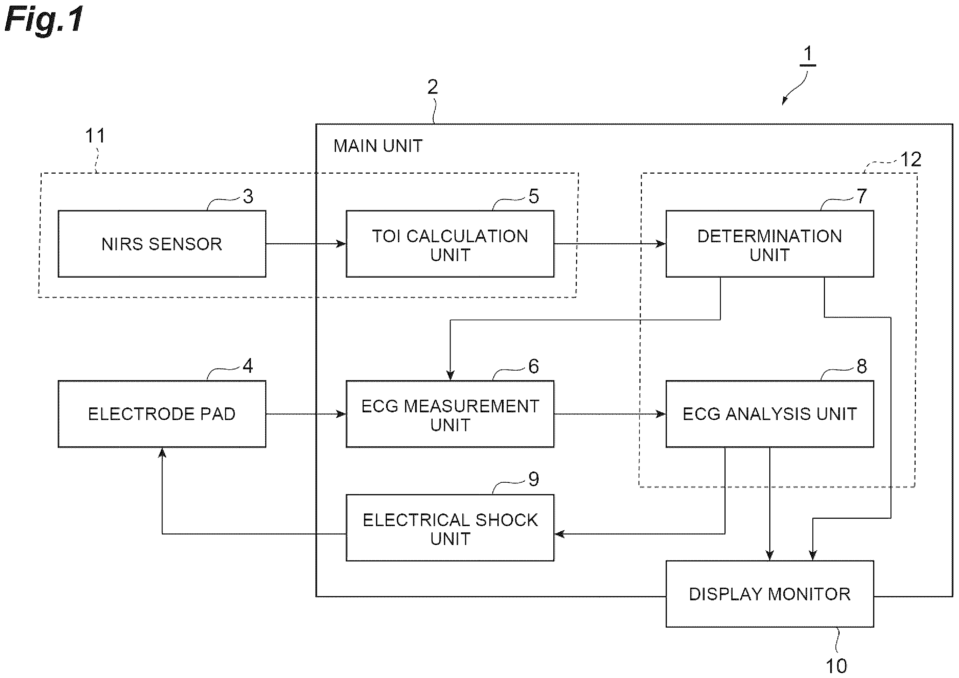

FIG. 1 is a functional block diagram illustrating a configuration of a defibrillator 1 according to an embodiment. The defibrillator 1 is an apparatus for performing defibrillation for a ventricular tachycardia (VT), a ventricular fibrillation (VF), an atrial fibrillation (AF), and the like of a patient. The term "defibrillation" as used herein refers to a treatment in which an abnormal electric signal path is cut off by an electrical stimulation to promote an improvement to a normal electric signal path.

The defibrillator 1 of the present embodiment includes a main unit 2, an NIRS sensor 3, and an electrode pad 4. The main unit 2 includes a TOI calculation unit 5, an ECG measurement unit 6, a determination unit 7, an ECG analysis unit 8, an electrical shock unit 9, and a display monitor 10.

The main unit 2 has a hollow housing, and houses the TOI calculation unit 5, the ECG measurement unit 6, the determination unit 7, the ECG analysis unit 8, and the electrical shock unit 9 in the housing. The display monitor 10 is attached to an outer surface of the housing, and is visible from the outside of the main unit 2. The NIRS sensor 3 and the electrode pad 4 are each attached to a tip of a cable extending from the main unit 2.

The NIRS sensor 3 and the TOI calculation unit 5 constitute a TOI measurement unit 11 in the present embodiment. The TOI measurement unit 11 measures a numerical value related to a tissue oxygen saturation (tissue oxygenation index: TOI) in a body (for example, head) of the patient, which varies due to repetition of chest compression, and displays the measurement result on the display monitor 10 to notify the person performing the chest compression.

Specifically, the TOI measurement unit 11 inputs near-infrared light into a predetermined light input position from the NIRS sensor 3 attached to a body surface, and detects an intensity of the near-infrared light output from a predetermined light detection position on the body surface. Thus, the TOI measurement unit 11 examines the influence of oxygenated hemoglobin (O₂Hb) and deoxygenated hemoglobin (HHb) on the near-infrared light, and repeatedly calculates respective concentrations of the oxygenated hemoglobin (O₂Hb) and the deoxygenated hemoglobin (HHb) based on the results. The above method is called near-infrared spectroscopy (NIRS).

The TOI is a numerical value representing the degree of oxygenation of blood hemoglobin, and is calculated as a ratio of an oxygenated hemoglobin concentration with respect to a total hemoglobin concentration. Further, in the present embodiment, the numerical value related to the TOI is the TOI itself, or a numerical value having a close correlation with the TOI. The numerical value calculated by the TOI measurement unit 11 in the present embodiment is the TOI itself, or a relative temporal change amount of the TOI based on the TOI at a certain timing as a reference.

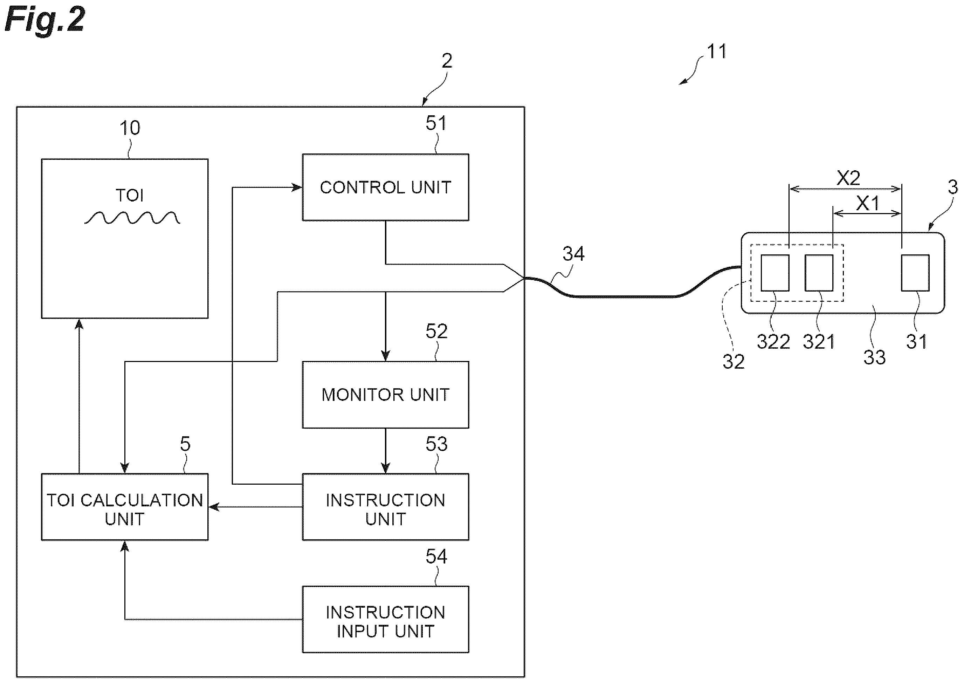

FIG. 2 is a diagram illustrating a specific configuration example of the TOI measurement unit 11. The NIRS sensor 3 is fixed, for example, by an adhesive tape, a stretchable band, or the like to a vicinity of a forehead without hair or a carotid artery.

The NIRS sensor 3 includes a light input unit 31 and a light detection unit 32. The light input unit 31 and the light detection unit 32 are disposed while being spaced from each other, and are substantially integrated by a holder 33 made of flexible black silicone rubber. The material of the holder 33 may be replaced with another material having flexibility equivalent to silicone rubber. The NIRS sensor 3 is electrically coupled to the main unit 2 via a cable 34.

The light input unit 31 receives a control signal transmitted from the main unit 2 and outputs measurement light of a predetermined wavelength. The measurement light is incident substantially perpendicularly on a skin layer of the body surface of the patient. The light input unit 31 includes, for example, a laser diode, a light emitting diode (LED), or a super luminescent diode (SLD), and a driving circuit for the diode. The wavelength of the measurement light is included in a near-infrared region, and in one example, the measurement light includes wavelength components of 735 nm, 810 nm, and 850 nm.

The light detection unit 32 generates a detection signal according to the intensity of the measurement light propagated inside the body of the patient and emitted from the body surface. The light detection unit 32 is, for example, a one-dimensional photosensor, and includes a plurality of photodetectors arranged in a distance direction from the light input unit 31. The light detection unit 32 of the present configuration example includes two photodetectors of a first photodetector 321 and a second photodetector 322.

The first photodetector 321 is provided at a position separated from the light input unit 31 by a first distance X1. The second photodetector 322 is provided at a position separated from the light input unit 31 by a second distance X2 (> X1). The distance X1 is, for example, 3 cm, and the distance X2 is, for example, 4 cm.

The first photodetector 321 generates a first detection signal according to the intensity of the received measurement light. The second photodetector 322 generates a second detection signal according to the intensity of the received measurement light. Each of the photodetectors 321 and 322 includes a semiconductor light receiving element such as a photodiode (PD) or an avalanche photodiode (APD), and a preamplifier which integrates and amplifies a current output from the semiconductor light receiving element.

In the above configuration, each of the photodetectors 321 and 322 can detect a weak current with high sensitivity to generate the detection signal, and transmit the detection signal to the main unit 2 via the cable 34. In addition, the light detection unit 32 may be a two-dimensional photosensor, and may be configured by, for example, a CCD image sensor or a CMOS image sensor.

The TOI measurement unit 11 includes a control unit 51, a monitor unit 52, and an instruction unit 53 in the main unit 2, in addition to the TOI calculation unit 5. The TOI measurement unit 11 includes a computer including an operation circuit such as a CPU and a storage device such as a memory. The TOI calculation unit 5, the control unit 51, the monitor unit 52, and the instruction unit 53 are implemented by the operation circuit reading and executing a program stored in advance in the storage device.

The control unit 51 is electrically coupled to the NIRS sensor 3 via the cable 34. The control unit 51 performs light output control of the light input unit 31 and light detection control of the photodetectors 321 and 322. That is, start and end of output of the measurement light in the light input unit 31 are performed based on the control signal from the control unit 51. Further, start and end of detection of the measurement light in the photodetectors 321 and 322 are performed based on the control signal from the control unit 51.

The control unit 51 causes the photodetectors 321 and 322 to start the detection of the measurement light before the attachment of the NIRS sensor 3 (standby mode). The standby mode continues until the TOI calculation unit 5 starts the calculation of the numerical value related to the TOI. The TOI calculation unit 5 does not perform the calculation of the numerical value related to the TOI in the standby mode.

The monitor unit 52 performs monitoring (observation) of changes of the light intensities input to the photodetectors 321 and 322 based on the detection signals from the light detection unit 32 which are continuously generated before the calculation start of the numerical value related to the TOI, that is, in the standby mode. The monitor unit 52 continuously outputs the monitoring result to the instruction unit 53.

The instruction unit 53 determines the calculation start of the numerical value related to the TOI based on the monitoring result obtained from the monitor unit 52, and instructs the TOI calculation unit 5 to start the calculation (measurement mode). The detection signal from each of the photodetectors 321 and 322 at the time of the standby mode start indicates the light intensity before the NIRS sensor 3 is attached to the body surface of the patient, that is, the intensity of ambient light in the measurement environment (for example, sunlight for outdoors, and illumination light for indoors). Then, when the NIRS sensor 3 is attached to the body surface of the patient, the intensity of the ambient light decreases to a predetermined ratio (for example, 10%) or less with respect to the intensity before the attachment.

The instruction unit 53 can know such a decrease in the intensity based on whether or not the intensity of the measurement light detected by the photodetectors 321 and 322 has changed to be within a predetermined range. Therefore, the instruction unit 53 may determine the start of the calculation on the condition that the intensity of the measurement light detected by the photodetectors 321 and 322 is within the predetermined range.

In addition, even before the NIRS sensor 3 is attached to the patient, when the NIRS sensor 3 is handled by the rescuer, the detection intensity of the ambient light may decrease, for example, by covering the light detection unit 32 with a hand or the like. Therefore, the instruction unit 53 may determine the start of the calculation on the condition that the state in which the intensity of the measurement light detected by the light detection unit 32 is within the predetermined range continues for a predetermined period (for example, three seconds). Further, the instruction unit 53 may determine the start of the calculation on the condition that the change of the intensity of the measurement light detected by the photodetectors 321 and 322 exceeds a predetermined width.

Further, the instruction unit 53 may also determine the start of the calculation based on the light intensity detected by the second photodetector 322, which is farther from the light source among the two photodetectors 321 and 322. In this case, it is possible to more accurately obtain the change in the input light intensity (ambient light intensity), and to accurately detect the timing at which the NIRS sensor 3 is attached to the body surface of the patient.

In addition, the defibrillator 1 may further include an instruction input unit 54 (for example, a button) for the rescuer to instruct the calculation start of the numerical value related to the TOI. In this case, the TOI calculation unit 5 starts the calculation of the numerical value related to the TOI based on an action of the start to the instruction input unit 54 such as pressing of the button.

The TOI calculation unit 5 is a calculation processing circuit electrically coupled to the NIRS sensor 3 via the cable 34, and is an example of the calculation unit in the present embodiment. The TOI calculation unit 5 calculates the numerical value related to the TOI in the body, which varies due to repetition of the chest compression, based on the detection signal from the light detection unit 32 according to the intensity of the measurement light propagated through the inside of the body of the patient. The TOI calculation unit 5 starts the calculation of the numerical value related to the TOI by receiving the signal from the instruction unit 53.

The TOI calculation unit 5 calculates the numerical value related to the TOI using a near-infrared spectroscopic method such as a spatially resolved spectroscopy method (SRS method), a modified Beer Lambert method (MBL method), a phase resolved spectroscopy method (PRS method), a time resolved spectroscopy method (TRS method), or a phase modulation spectroscopy method (PMS method).

The MBL method uses the fact that the detected light amount changes according to the hemoglobin concentration (oxygenated hemoglobin concentration and deoxygenated hemoglobin concentration) in the measurement object. A relative change amount of the oxygenated hemoglobin concentration, a relative change amount of the deoxygenated hemoglobin concentration, and a relative change amount of the total hemoglobin concentration can be obtained by the MBL method. In the MBL method, the detection signal from any one of the photodetectors 321 and 322 is used. The SRS method uses the fact that the detected light amount changes according to distances between the light input unit 31 and the photodetectors 321 and 322. In the SRS method, the detection signals from both the photodetectors 321 and 322 are used.

The TRS method uses the fact that the time width of input pulsed measurement light becomes longer due to hemoglobin present in the measurement object. More specifically, an absolute value of the hemoglobin concentration is obtained by fitting simulation data based on a temporal spread of the pulsed measurement light and a temporal spread of the detected light. In the PRS method, modulated measurement light is input to the measurement object, and an absolute value of the hemoglobin concentration is obtained by obtaining a phase difference between the measurement light before the input and the measurement light after the detection. Therefore, according to the TRS method or the PRS method, not a relative value but an absolute value of the TOI can be obtained.

It is preferable that the calculation cycle of the numerical value related to the TOI is 0.2 seconds or less (5 Hz or more in terms of a calculation frequency). In general, it is said that a preferable cycle of the chest compression is about 100 times per minute (that is, once per 0.6 seconds) or more. Further, when the calculation cycle of the relative change amount is one third or less of the above cycle, it is possible to suitably detect the concentration change caused by the chest compression. Further, it is preferable that the predetermined frequency is 1.66 Hz or less. In this case, it is possible to suitably extract information on the concentration change due to the chest compression of about 100 times per minute or more.

The display monitor 10 displays information on the calculation result of the numerical value related to the TOI sent from the TOI calculation unit 5. The rescuer can determine whether the chest compression is appropriately performed with reference to the display of the display monitor 10.

FIG. 1 is referred again. The ECG measurement unit 6 measures an electrocardiogram (ECG) of the patient in order to determine whether an electrical shock is required for the patient. The ECG measurement unit 6 is electrically coupled to the electrode pad 4, and records the state of the electrical activity of the heart in time series by temporally continuously detecting the electric signals from the electrode pad 4 which is attached to the patient. In addition, the electrode pad 4 includes a positive electrode pad and a negative electrode pad, which are attached to different positions near the chest.

The determination unit 7 and the ECG analysis unit 8 constitute a control unit 12. The control unit 12 includes a computer including an operation circuit such as a CPU and a storage device such as a memory. The determination unit 7 and the ECG analysis unit 8 are implemented by the operation circuit reading and executing a program stored in advance in the storage device. In addition, the computer which implements the determination unit 7 and the ECG analysis unit 8 may be configured in common with the computer which implements the TOI calculation unit 5 and the like of the TOI measurement unit 11.

The determination unit 7 determines whether or not the numerical value relating to the TOI acquired in the TOI measurement unit 11 exceeds a predetermined threshold value. The determination unit 7 provides a signal for starting (or enabling to start) the measurement of the electrocardiogram to the ECG measurement unit 6 on the condition that the numerical value related to the TOI exceeds the predetermined threshold value.

The threshold value may be, for example, a value included in a range of 40% to 60% in terms of the temporal relative change amount of the TOI, and is more preferably, a value included in a range of 45% to 50% in terms of the temporal relative change amount of the TOI. The threshold value is stored in advance in the storage device.

When the TOI exceeds the threshold value, the determination unit 7 provides a display signal for informing the rescuer that the measurement of the electrocardiogram is started to the display monitor 10. Upon receiving the display signal, the display monitor 10 displays a character or a pattern (for example, "electrocardiogram analysis is being performed" or the like) indicating that the measurement of the electrocardiogram is started on the screen to notify the rescuer.

When the TOI does not exceed the threshold value, the determination unit 7 provides a display signal for informing the rescuer that the chest compression for the patient should be continued to the display monitor 10. Upon receiving the display signal, the display monitor 10 displays a character or a pattern (for example, "continue cardiac massage" or the like) indicating that the chest compression should be continued on the screen to notify the rescuer.

In addition, the main unit 2 may include a speaker, which is not shown. In this case, when the TOI exceeds the threshold value, the determination unit 7 may provide a signal for informing the rescuer that the measurement of the electrocardiogram is started to the speaker, and upon receiving the signal, the speaker may output a sound or the like indicating that the measurement of the electrocardiogram is started.

Further, when the TOI does not exceed the threshold value, the determination unit 7 may provide a signal for informing the rescuer that the chest compression for the patient should be continued to the speaker, and upon receiving the signal, the speaker may output a sound or the like indicating that the chest compression should be continued. The display monitor 10 and the speaker are examples of the information notification unit in the present embodiment.

The ECG analysis unit 8 acquires the measurement result of the electrocardiogram from the ECG measurement unit 6. The ECG analysis unit 8 analyzes the electrocardiogram, and determines whether the electrical shock is required for the patient. When it is determined that the electrical shock is not required, the ECG analysis unit 8 provides a display signal for informing the rescuer that the electrical shock is not required to the display monitor 10. Upon receiving the display signal, the display monitor 10 displays a character, a symbol, or the like indicating that the chest compression should be continued on the screen to notify the rescuer.

In addition, when the main unit 2 includes the speaker, when the electrical shock is not required, the ECG analysis unit 8 may provide a signal for informing the rescuer that the chest compression for the patient should be continued to the speaker, and upon receiving the signal, the speaker may output a sound or the like indicating that the chest compression should be continued.

Further, when it is determined that the electrical shock is required, the ECG analysis unit 8 provides a signal for starting the electrical shock to the electrical shock unit 9. Upon receiving the signal from the ECG analysis unit 8, the electrical shock unit 9 outputs an electrical energy for the electrical shock from the electrode pad 4. In addition, the electrode pad used for the electrical shock may be configured in common with the electrode pad used for the ECG measurement, or may be provided separately from the electrode pad used for the ECG measurement.

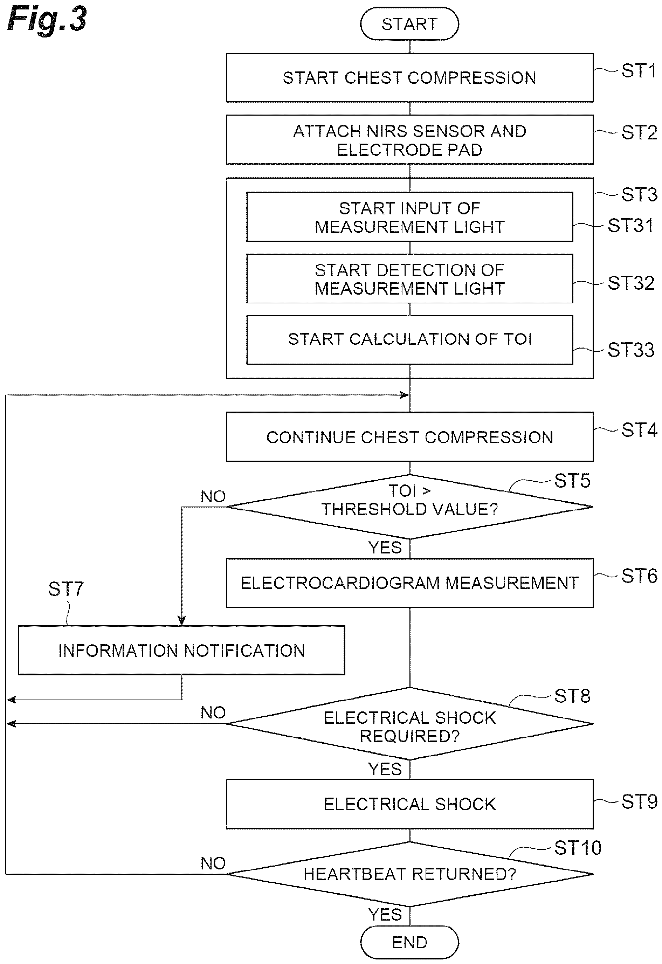

Next, a method for defibrillation according to the present embodiment will be described together with an operation method of the defibrillator 1. FIG. 3 is a flowchart illustrating the operation method of the defibrillator 1 and the method for defibrillation.

First, the rescuer starts the chest compression after confirming that there is no response of the patient and there is no breathing (step ST1). Next, the rescuer attaches the NIRS sensor 3 and the electrode pad 4 of the defibrillator 1 to the patient (step ST2). At this time, the monitor unit 52 detects attachment of the NIRS sensor 3, and the TOI measurement unit 11 starts acquisition of the numerical value related to the TOI (step ST3).

Specifically, the light input unit 31 starts input of the measurement light into the body of the patient (light input step ST31). Further, the light detection unit 32 starts detection of the measurement light propagated in the body of the patient and starts generation of the detection signal according to the intensity of the measurement light (light detection step ST32). Further, the TOI calculation unit 5 starts calculation of the numerical value related to the TOI based on the detection signal (calculation step ST33).

Further, the TOI calculation unit 5 may start calculation of the numerical value related to the TOI by an action of start by the input (for example, pressing of the button) from the instruction input unit 54. Thus, in the situation where the TOI measurement unit 11 is continuously measuring the numerical value related to the TOI, the rescuer continues the chest compression (step ST4).

When the rescuer performs the operation input for performing the electrical shock, the determination unit 7 determines whether or not the numerical value related to the TOI exceeds the threshold value (step ST5). Further, when the numerical value related to the TOI exceeds the threshold value (step ST5: YES), the ECG measurement unit 6 starts measurement of the electrocardiogram (step ST6).

Further, when the numerical value related to the TOI does not exceed the threshold value (step ST5: NO), it notifies the rescuer that the chest compression for the patient should be continued through the display on the display monitor 10 and the sound of the speaker (information notification step ST7). Thereafter, the process returns to the step ST4, and the steps ST4 and ST5 are repeated. As described above, the threshold value may be any value included in the range of 40% to 60% in terms of TOI, and more preferably any value included in the range of 45% to 50% in terms of TOI.

After the electrocardiogram is measured in the step ST6, the ECG analysis unit 8 analyzes the electrocardiogram to determine whether the electrical shock is required (step ST8). When it is determined that the electrical shock is required (step ST8: YES), the electrical shock unit 9 outputs the electrical energy for the electrical shock from the electrode pad 4 (electrical shock step ST9). Further, when it is determined that the electrical shock is not required (step ST8: NO), the process returns to the step ST4, and the steps ST4 to ST8 are repeated.

After performing the electrical shock in the step ST9, the rescuer determines whether the heartbeat of the patient is returned (step ST10). When the heartbeat of the patient is not returned (step ST 10: NO), the process returns to the step ST4, and the steps ST4 to ST10 are repeated. When the heartbeat of the patient is returned (step ST10: YES), the treatment for the cardio pulmonary resuscitation is ended.

Effects obtained by the defibrillator 1 and the method for defibrillation of the present embodiment described above will be described.

As described above, in the cardio pulmonary resuscitation, the electrical shock is applied for removing an arrhythmia such as a ventricular fibrillation (VF) of the patient. Before the electrical shock is applied, the electrocardiogram analysis is performed in order to confirm the state of the arrhythmia of the patient.

However, the electrocardiogram analysis and charging for the electrical shock require a certain amount of time, and further, the chest compression cannot be performed when the electrocardiogram analysis and the charging are performed. Therefore, the blood oxygen concentration once increased by the chest compression gradually decreases during the electrocardiogram analysis and the charging. Thus, when the electrocardiogram analysis and the electrical shock are repeated, the blood oxygen concentration increased by the chest compression is decreased each time.

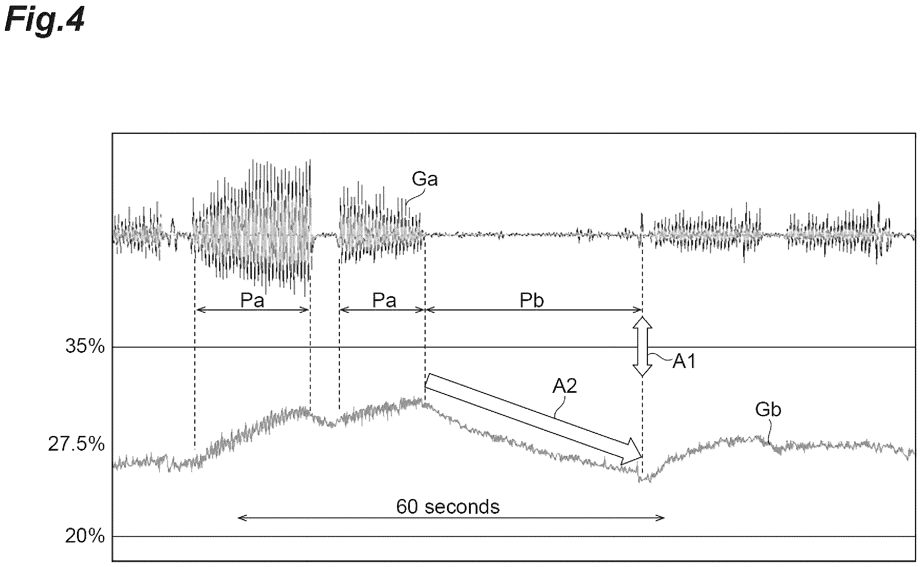

FIG. 4 to FIG. 7 are graphs showing results obtained by actually measuring the temporal relative change amount of the TOI in the cardio pulmonary resuscitation. In each of the figures, an upper graph Ga represents the temporal relative change amount of the oxygenated hemoglobin concentration in the head of the patient, and the vibration part included in the graph Ga indicates that the chest compression is performed or there is a spontaneous heartbeat. A lower graph Gb represents the temporal relative change amount of the TOI (unit: %) in the head of the patient.

A period Pa is a period in which the chest compression is performed. A period Pb is a period in which the electrocardiogram analysis and the charging for the electrical shock are performed. An arrow A1 indicates a timing at which the electrical shock is performed. In addition, in the graphs, the horizontal axis indicates the time. Further, scales of the time axes in FIG. 4 to FIG. 7 are different from each other.

Referring to FIG. 4, the TOI gradually increases in the period Pa in which the chest compression is performed, but the TOI starts to decrease immediately after entering the period Pb in which the electrocardiogram analysis is performed. Further, during the period Pb, the TOI continues to decrease (arrow A2 in the figure). The above decrease of the TOI is caused by stopping the chest compression while performing the electrocardiogram analysis.

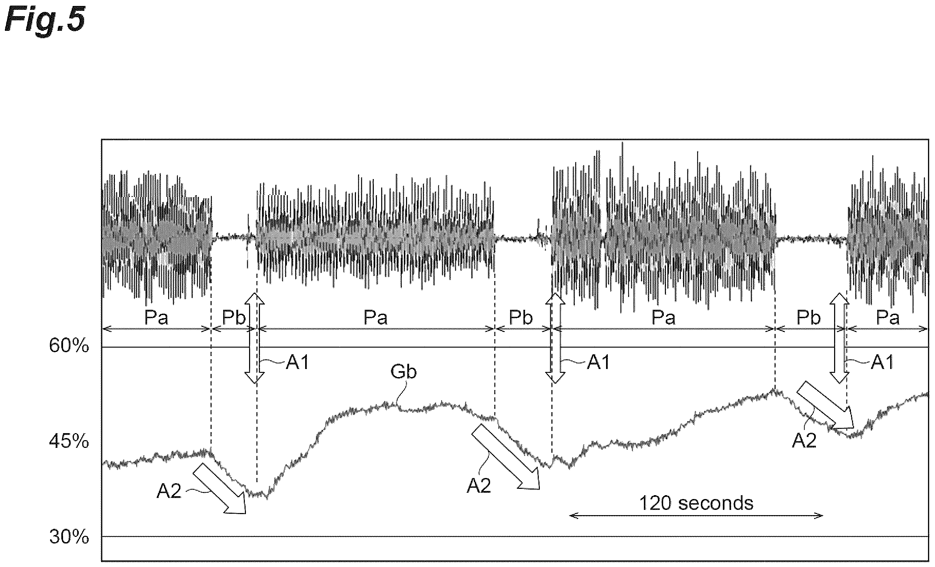

Further, referring to FIG. 5, it can be seen that even when the TOI is increased by the chest compression (period Pa), when the electrocardiogram analysis (period Pb) is performed a plurality of times in the middle thereof, the TOI is decreased each time, and much of the increase in the TOI due to the chest compression is canceled. As a result, the increase of the TOI becomes slow, which causes the cardio pulmonary resuscitation to take time.

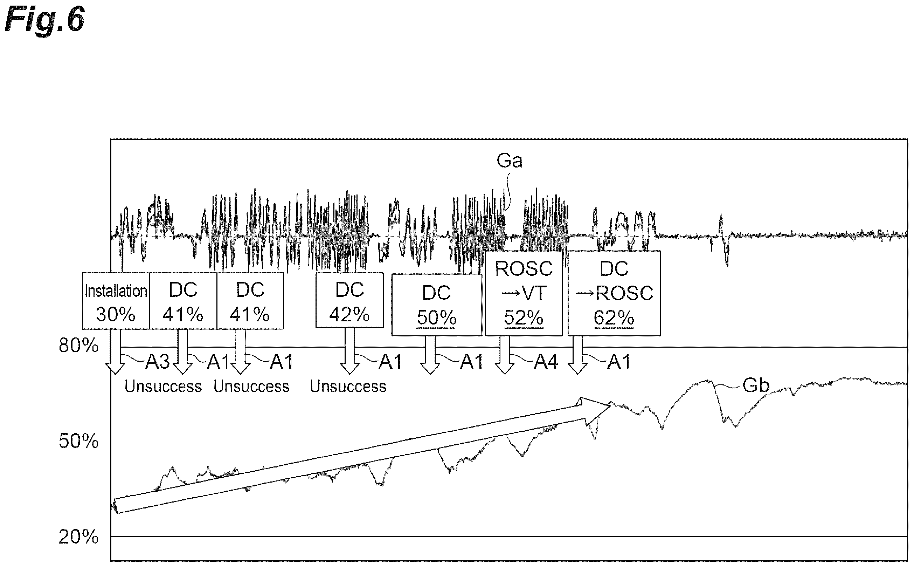

With respect to the above problem, the present inventors have found that the magnitude of the TOI of the patient affects success or failure of the defibrillation by the electrical shock. FIG. 6 shows the example of successful defibrillation for refractory VF.

In the example shown in FIG. 6, after the defibrillator 1 is installed and the measurement of the TOI is started (arrow A3), the electrical shock (DC) is performed a plurality of times. In addition, a numerical value in a frame in the figure indicates a value of the TOI at each timing. Among them, the numerical value in the frame at the time of the electrical shock (DC) indicates the value of the TOI immediately before the electrocardiogram analysis. After the first return of spontaneous circulation (ROSC), the ventricular fibrillation (VF) occurs again (arrow A4), and then, the resuscitation is successful by the second return of spontaneous circulation (ROSC) by the electrical shock (DC).

Referring to the example shown in FIG. 6, the temporal relative change amount of the TOI is 30% when the defibrillator 1 is installed, and the temporal relative change amount of the TOI gradually increases due to the chest compression thereafter. The TOI before the electrocardiogram analysis in the first to third electrical shocks (DC) is 41% to 42%, and the return of spontaneous circulation (ROSC) is unsuccessful in these electrical shocks. However, the temporal relative change amount of the TOI before the electrocardiogram analysis in the fourth electrical shock (DC) is 50%, and the first return of spontaneous circulation (ROSC) is successful. Further, the temporal relative change amount of the TOI before the electrocardiogram analysis in the fifth electrical shock (DC) after returning to the ventricular fibrillation (VF) is 62%, and the second return of spontaneous circulation (ROSC) is successful.

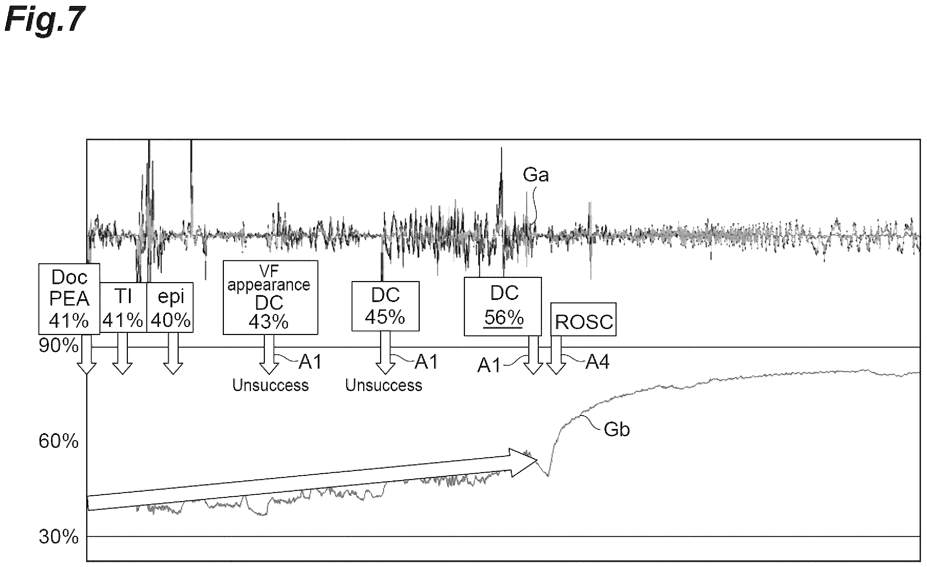

In the example shown in FIG. 7, after pulseless electrical activity (PEA), the ventricular fibrillation (VF) appears through tracheal intubation (TI) and epinephrine administration (epi), and the first electrical shock (DC) is performed. Thereafter, the second electrical shock (DC) is performed, but both the first and second returns of spontaneous circulation (ROSC) are unsuccessful. Thereafter, the return of spontaneous circulation (ROSC) is realized by the third electrical shock (DC), and the resuscitation is successful. The temporal relative change amounts of the TOI before the electrocardiogram analysis in the first and second electrical shocks (DC) are 43% and 45%, respectively, and the temporal relative change amount of the TOI before the electrocardiogram analysis in the third electrical shock (DC) is 56%.

According to the examples shown in FIG. 6 and FIG. 7, it can be seen that, when the electrical shock is performed after the TOI of the patient is increased by the chest compression, a success rate of the defibrillation is significantly improved. In these examples, all the electrical shocks performed when the temporal relative change amount of the TOI is 45% or less are unsuccessful, and all the electrical shocks performed when the temporal relative change amount of the TOI is 50% or more are successful.

Therefore, for example, the threshold value is set within a range of 45% to 50%, and the electrocardiogram analysis and the electrical shock are performed on the condition that the temporal relative change amount of the TOI exceeds the threshold value, and when the temporal relative change amount of the TOI does not exceed the threshold value, the electrocardiogram analysis and the electrical shock are not performed and the chest compression is preferentially performed. In this case, the success rate of the defibrillation can be increased, and the number of times of the electrocardiogram analysis and the electrical shock can be reduced.

In addition, the TOI in FIG. 6 and FIG. 7 is a temporal relative value, and thus, it is considered to vary according to a surrounding environment, an age of the patient, a body shape of the patient, and the like. Therefore, the numerical value range of 45% to 50% described above is merely an example, and a numerical value outside the above range may be set as the threshold value in some cases.

Even in the above case, when the threshold value is included in a range of 40% to 60%, the success rate of the defibrillation can be generally increased, in consideration of a large amount of data of the present inventors. Further, even when the TOI to be acquired is the temporal relative value, it is possible to set a generally preferable threshold value by acquiring data on a relationship between the temporal relative value of the TOI and success or failure of the defibrillation from many patients.

In the present embodiment, the electrocardiogram of the patient is measured in the ECG measurement unit 6 (step ST6) on the condition that the TOI acquired in the TOI measurement unit 11 (step ST3) exceeds the threshold value. Therefore, the electrocardiogram analysis and the electrical shock may be performed when the TOI exceeds the threshold value, and the chest compression may be continued when the TOI does not exceed the threshold value, and thus, it is possible to increase the success rate of the defibrillation by the electrical shock, and reduce the number of times of the electrocardiogram analysis and the electrical shock.

As in the present embodiment, the TOI measurement unit 11 may include the light input unit 31 for inputting the measurement light into the body of the patient, the light detection unit 32 for detecting the measurement light propagated in the body of the patient and generating the detection signal according to the intensity of the measurement light, and the TOI calculation unit 5 for calculating the numerical value related to the TOI based on the detection signal. Further, the step ST3 of starting acquisition of the numerical value related to the TOI may include the light input step ST31 of starting input of the measurement light into the body of the patient, the light detection step ST32 of starting detection of the measurement light propagated in the body of the patient and starting generation of the detection signal according to the intensity of the measurement light, and the calculation step ST33 of starting calculation of the numerical value related to the TOI based on the detection signal. In this case, the numerical value related to the TOI of the patient can be non-invasively and easily measured, and thus, the burden on the patient and the rescuer can be reduced.

As in the present embodiment, the electrical shock unit 9 may output the electrical energy for the electrical shock from the electrode pad 4 when it is determined that the electrical shock is required for the patient based on the measurement result of the electrocardiogram by the ECG measurement unit 6. Further, the electrical energy for the electrical shock may be output from the electrode pad 4 in the electrical shock step ST9 when it is determined that the electrical shock is required for the patient in the step ST8. In this case, the electrical shock for the defibrillation can be suitably performed.

As in the present embodiment, the display monitor 10 may notify the rescuer that the chest compression for the patient should be continued when the numerical value related to the TOI does not exceed the threshold value. Further, the information notification step ST7 may notify the rescuer that the chest compression for the patient should be continued when the numerical value related to the TOI does not exceed the threshold value. In this case, when the TOI does not exceed the threshold value, the rescuer is prompted to continue the chest compression, and thus, the decrease of the TOI can be suppressed.

As in the present embodiment, the threshold value may be a value included in the range of 40% to 60% in terms of the temporal relative change amount of the TOI. Further, the threshold value may be a value included in the range of 45% to 50% in terms of the temporal relative change amount of the TOI. According to the findings of the present inventors, for example when a certain threshold value within the above range is exceeded, the success rate of the defibrillation by the electrical shock is significantly increased.

The apparatus and the method for the defibrillation are not limited to the embodiments and configuration examples described above, and may be modified in various ways. For example, in the above embodiment, near-infrared spectroscopy is exemplified as the measurement method of the TOI, and the measurement method of the TOI is not limited thereto, and various other methods can be used.

The apparatus for defibrillation of the above embodiment includes an oxygen saturation measurement unit for acquiring a numerical value related to an oxygen saturation of a patient; an electrocardiogram measurement unit for measuring an electrocardiogram of the patient in order to determine whether an electrical shock is required for the patient; and a control unit for starting measurement of the electrocardiogram of the patient in the electrocardiogram measurement unit on the condition that the numerical value acquired in the oxygen saturation measurement unit exceeds a threshold value.

The method for defibrillation of the above embodiment includes a step of starting acquisition of a numerical value related to an oxygen saturation of a patient; a step of measuring an electrocardiogram of the patient on the condition that the numerical value related to the oxygen saturation exceeds a threshold value; and a step of determining whether an electrical shock is required for the patient based on a measurement result of the electrocardiogram.

In the above apparatus, the oxygen saturation measurement unit may include a light input unit for inputting measurement light into a body of the patient; a light detection unit for detecting the measurement light propagated in the body of the patient, and generating a detection signal according to an intensity of the measurement light; and a calculation unit for calculating the numerical value related to the oxygen saturation based on the detection signal.

In the above method, the step of starting the acquisition of the numerical value related to the oxygen saturation may include a light input step of starting input of measurement light into a body of the patient; a light detection step of starting detection of the measurement light propagated in the body of the patient, and starting generation of a detection signal according to an intensity of the measurement light; and a calculation step of starting calculation of the numerical value related to the oxygen saturation based on the detection signal.

According to the above apparatus and the method, the numerical value related to the oxygen saturation of the patient can be non-invasively and easily measured, and thus, a burden on the patient and the rescuer can be reduced.

The above apparatus may further include an electrical shock unit, and the electrical shock unit may output an electrical energy for the electrical shock from an electrode when it is determined that the electrical shock is required for the patient based on a measurement result of the electrocardiogram by the electrocardiogram measurement unit. The above method may further include an electrical shock step, and an electrical energy for the electrical shock may be output from an electrode in the electrical shock step when it is determined that the electrical shock is required for the patient in the step of determining. In this case, the electrical shock for the defibrillation can be suitably performed.

The above apparatus may further include an information notification unit, and the information notification unit may notify a rescuer that chest compression for the patient should be continued when the numerical value related to the oxygen saturation does not exceed the threshold value. The above method may further include an information notification step, and the information notification step may notify a rescuer that chest compression for the patient should be continued when the numerical value related to the oxygen saturation does not exceed the threshold value. In this case, when the oxygen saturation does not exceed the threshold value, the rescuer is prompted to continue the chest compression, and thus, a decrease of the oxygen saturation can be suppressed.

In the above apparatus and the method, the threshold value may be any value included in a range of 40% to 60% in terms of a temporal relative change amount of the oxygen saturation. Further, the threshold value may be any value included in a range of 45% to 50% in terms of the temporal relative change amount of the oxygen saturation. According to the findings of the present inventors, for example when the threshold value within the above range is exceeded, the success rate of the defibrillation by the electrical shock is significantly increased.

### Industrial Applicability

The embodiments can be used as an apparatus and a method for defibrillation capable of increasing a success rate of defibrillation by an electrical shock and reducing the number of times of electrocardiogram analysis and the electrical shock.

### Reference Signs List

1 - defibrillator, 2 - main unit, 3 - NIRS sensor, 4 - electrode pad, 5 - TOI calculation unit, 6 - ECG measurement unit, 7 - determination unit, 8 - ECG analysis unit, 9 - electrical shock unit, 10 - display monitor, 11 - TOI measurement unit, 12 - control unit, 31 - light input unit, 32 - light detection unit, 33 - holder, 34 - cable, 51 - control unit, 52 - monitor unit, 53 - instruction unit, 54 - instruction input unit, 321, 322 - photodetector.

## Claims

1. An apparatus for defibrillation comprising:
an oxygen saturation measurement unit for acquiring a numerical value related to an oxygen saturation of a patient;
an electrocardiogram measurement unit for measuring an electrocardiogram of the patient in order to determine whether an electrical shock is required for the patient; and
a control unit for starting measurement of the electrocardiogram of the patient in the electrocardiogram measurement unit on the condition that the numerical value acquired in the oxygen saturation measurement unit exceeds a threshold value.

2. The apparatus according to Claim 1, wherein the oxygen saturation measurement unit includes:
a light input unit for inputting measurement light into a body of the patient;
a light detection unit for detecting the measurement light propagated in the body of the patient, and generating a detection signal according to an intensity of the measurement light; and
a calculation unit for calculating the numerical value related to the oxygen saturation based on the detection signal.

3. The apparatus according to Claim 1 or 2, further comprising an electrical shock unit, wherein
the electrical shock unit outputs an electrical energy for the electrical shock from an electrode when it is determined that the electrical shock is required for the patient based on a measurement result of the electrocardiogram by the electrocardiogram measurement unit.

4. The apparatus according to any one of Claims 1 to 3, further comprising an information notification unit, wherein
the information notification unit notifies a rescuer that chest compression for the patient should be continued when the numerical value acquired by the oxygen saturation measurement unit does not exceed the threshold value.

5. The apparatus according to any one of Claims 1 to 4, wherein the threshold value is a value included in a range of 40% to 60% in terms of a temporal relative change amount of the oxygen saturation.

6. The apparatus according to Claim 5, wherein the threshold value is a value included in a range of 45% to 50% in terms of the temporal relative change amount of the oxygen saturation.

7. A method for defibrillation comprising:
a step of starting acquisition of a numerical value related to an oxygen saturation of a patient;
a step of measuring an electrocardiogram of the patient on the condition that the numerical value related to the oxygen saturation exceeds a threshold value; and
a step of determining whether an electrical shock is required for the patient based on a measurement result of the electrocardiogram.

8. The method according to Claim 7, wherein the step of starting the acquisition of the numerical value related to the oxygen saturation includes:
a light input step of starting input of measurement light into a body of the patient;
a light detection step of starting detection of the measurement light propagated in the body of the patient, and starting generation of a detection signal according to an intensity of the measurement light; and
a calculation step of starting calculation of the numerical value related to the oxygen saturation based on the detection signal.

9. The method according to Claim 7 or 8, further comprising an electrical shock step, wherein
an electrical energy for the electrical shock is output from an electrode in the electrical shock step when it is determined that the electrical shock is required for the patient in the step of determining.

10. The method according to any one of Claims 7 to 9, further comprising an information notification step, wherein
the information notification step notifies a rescuer that chest compression for the patient should be continued when the numerical value related to the oxygen saturation does not exceed the threshold value.

11. The method according to any one of Claims 7 to 10, wherein the threshold value is a value included in a range of 40% to 60% in terms of a temporal relative change amount of the oxygen saturation.

12. The method according to Claim 11, wherein the threshold value is a value included in a range of 45% to 50% in terms of the temporal relative change amount of the oxygen saturation.
